# EUROPÄISCHE PATENTSCHRIFT

(11) **EP 0 547 422 B1**
(45) Veröffentlichungstag und Bekanntmachung des Hinweises auf die Patenterteilung: **13.03.1996**
(21) Anmeldenummer: 92120456.6
(22) Anmeldetag: 01.12.1992
(51) Int. Cl.: A61K 9/14, A61K 9/50, A61K 47/18

(54) **Stabile pulverförmige Vitamin- und/oder Carotinoid-Präparate und Verfahren zu deren Herstellung**
Stable powdery vitamin and/or carotinoid preparations and process for their production
Compositions des vitamines et/ou carotinoides stables en poudre et procédé de leur préparation

(30) Priorität: 14.12.1991 DE 4141351
(43) Veröffentlichungstag der Anmeldung: 23.06.1993
(73) Patentinhaber: BASF Aktiengesellschaft, D-67063 Ludwigshafen (DE)
(72) Erfinder: Schneider, Joachim U., Dr., W-6719 Weisenheim (DE); Schumacher, Horst, Dr., W-6719 Bobenheim (DE); Bewert, Wolfgang, Dr., W-6710 Frankenthal (DE); Gaus, Guenter, Dr., W-6843 Biblis (DE); Rheude, Udo, Dr., W-6900 Heidelberg (DE)

(56) Entgegenhaltungen:
- EP-A- 0 140 255
- EP-A- 0 285 682
- US-A- 4 863 950

## Beschreibung

Die vorliegende Erfindung betrifft stabile heißwasserunlösliche Trockenpulver, bei welchen ein oder mehrere fettlösliche Vitamine und/oder ein oder mehrere Carotinoide in eine Matrix auf Basis von Gelatine eingebettet sind, erhältlich durch
a) Herstellung einer Dispersion enthaltend im wesentlichen diese fettlöslichen Wirkstoffe, reduzierende Zucker und als filmbildendes Kolloid Gelatine in Mengen von 20 bis 35, vorzugsweise 24 bis 34, inbesondere 25 bis 30 Gew.-%, bezogen auf die Trockenmasse des Pulvers, in Kombination mit einer oder mehreren physiologisch verträglichen organischen Aminoverbindungen, die eine basische primäre Aminogruppe und zusätzlich entweder eine weitere Aminogruppe, eine Hydroxylgruppe, eine Alkoxygruppe oder eine Carboxylgruppe enthalten, in freier oder in salzartig gebundener Form und/oder in Kombination mit soviel einer basischen Alkali- oder Erdalkaliverbindung, daß die Dispersion einen pH-Wert von 7,5 bis 10, vorzugsweise 8 bis 9,5 aufweist,
b) Überführung dieser Dispersion in trockene, pulverförmige Vitamin- und/oder Carotinoidpräparate und
c) thermische Härtung des Pulvers bei Temperaturen von 60 bis 180°C, vorzugsweise 70 bis 130°C, sowie ein Verfahren zu deren Herstellung.

Pulverförmige Vitamin- und Carotinoid-Präparate sind allgemein bekannt und werden in der pharmazeutischen Industrie sowie in der Nahrungs- und Futtermittelindustrie in großem Umfang verwendet. So sind in der Literatur viele Verfahren zur Herstellung geeigneter Präparate beschrieben.

Üblicherweise dispergiert man die fettlöslichen Vitamine und/oder Carotinoide in einer wäßrigen Lösung eines organischen filmbildenden Kolloids und überführt die erhaltene Dispersion letztlich in trockene pulverförmige Präparate.

Als filmbildendes Kolloid wird nach dem Stand der Technik üblicherweise Gelatine verwendet. Als Gründe für die Bevorzugung der Gelatine sind zu nennen:
1) Gelatine ist ein ausgezeichnetes dispersionsstabilisierendes und filmbildendes Mittel;
2) Gelatine bildet thermoreversible Gele, wodurch technisch mit geeigneten Verfahren ein Trocknen der Tröpfchen oder Beadlets der Dispersion ermöglicht und dadurch Partikel mit optimaler Teilchengröße erzielt werden;
3) Gelatine ist ein gutes Schutzkolloid und hat in Kombination mit Antioxidantien einen stabilisierenden Effekt, d.h. der Schutzfilm ist besonders undurchlässig für Sauerstoff, was für die besonders gegen Sauerstoff empfindlichen fettlöslichen Vitamine von besonderer Bedeutung ist (vgl. R.A. Morton in "Fatsoluble Vitamins", International Enzyclopaedia of Food and Nutrition, Vol. 9, Pergamon Press Ltd., 1970, S. 129f).

Besonders hohe Ansprüche werden an die Stabilität solcher Präparate gestellt, wenn diese als Zusatz zu Lebensmitteln oder zu Tierfutter verwendet werden sollen, da sie bei dieser Verwendung vielfach Einflüssen, wie erhöhten Temperaturen, Feuchtigkeit, mechanische Reibung oder Druck ausgesetzt sind, die für die empfindlichen Vitamine und Carotinoide äußerst schädlich sind. Es hat daher nicht an Versuchen gefehlt, Verfahren zu entwickeln, die thermisch und mechanisch besonders stabile Präparate liefern.

So ist es beispielsweise aus GB 993 138 bekannt, bestimmte, Gelatine als Matrix enthaltende, Vitaminpräparate dadurch zu stabilisieren, daß man die Partikel mit einem Gelatine-denaturierenden Mittel, wie Formaldehyd, Glyoxal, Acetaldehyd oder Dihydroxyaceton, behandelt und anschließend erhitzt oder aber nur einer Wärmebehandlung unterzieht.

Aus dem kürzlich erschienenen Patent EP-B1 285 682 ist ein Verfahren zur Herstellung von kugelförmigen Präparaten, welche fettlösliche Vitamine enthalten, durch Emulsionsbildung mittels Wasser, Gelatine und einem Zucker, Umwandlung der Emulsion in Tröpfchen, Sammeln der Tröpfchen in einer Masse aus Stärkepulver in einer Art, daß die Tröpfchen voneinander getrennt bleiben, bis sich ihre Form dauerhaft ausgebildet hat, Trennen der erhaltenen Partikel von überschüssigem Stärkepulver und anschließende Wärmebehandlung bei Temperaturen von 90 bis 180°C bekannt. Nachteilig an dem an sich recht guten Verfahren ist, daß zur Herstellung der Präparate größere Mengen an Gelatine (35 bis 45 Gew.-%, bezogen auf die Trockenmasse) benötigt werden, wodurch die technische Herstellung der Produkte hinsichtlich der Einsatzstoffkosten und der Verfahrensführung unwirtschaftlich wird.

Es war daher die Aufgabe der Erfindung, pulverförmige Vitamin- und/oder Carotinoid-Präparate herzustellen, die bei geringerem Gehalt an der teuren Gelatine eine mindestens gleichwertige Stabilität gegenüber hydrothermalen und mechanischen Belastungen zeigen und dadurch für die Herstellung von z.B. Prämixen, Pellets und Extrudaten für den Tierfutterbereich bzw. Tabletten für den Pharmabereich noch besser geeignet sind.

Gegenstand der Erfindung ist daher auch ein Verfahren, nach dem die oben definierten stabilen heißwasserunlöslichen Trockenpulver, welche ein oder mehrere fettlösliche Vitamine und/oder ein oder mehrere Carotinoide enthalten, erhältlich sind und welches folgende Verfahrensschritte beinhaltet:
a) Herstellung einer wäßrigen Dispersion, enthaltend im wesentlichen diese fettlöslichen Wirkstoffe, filmbildende Kolloide und reduzierende Zucker,
b) Überführung dieser Dispersion in trockene, pulverförmige Vitamin- und/oder Carotinoidpräparate, vorzugsweise durch Versprühen in eine Wolke aus einem Gas und hydrophober Kieselsäure, und
c) thermische Härtung des Pulvers bei Temperaturen von 60 bis 180°C,
und welches dadurch gekennzeichnet ist, daß man als filmbildendes Kolloid Gelatine in Mengen von 20 bis 35 Gew.-%, bezogen auf die Trockenmasse des Pulvers in Kombination mit einer oder mehreren freien oder salzartig gebundenen physiologisch verträglichen organischen Aminoverbindungen, die eine basische primäre Aminogruppe und zusätzlich entweder eine weitere Aminogruppe, eine Hydroxylgruppe, eine Alkoxylgruppe oder eine Carboxylgruppe enthalten, in freier oder salzartig gebundener Form und/oder in Kombination mit soviel einer basischen Alkali- oder Erdalkaliverbindung verwendet, daß die Dispersion einen pH-Wert von 7,5 bis 10 aufweist.

Mit Vorteil arbeitet man nach dem erfindungsgemäßen Verfahren so, daß man die Gelatine in Mengen von 24 bis 34 Gew.-%, insbesondere 25 bis 30 Gew.-%, bezogen auf die Trockenmasse des Pulvers in Kombination mit 0,3 bis 20 Gew.-%, vorzugsweise 0,5 bis 10 Gew.-%, bezogen auf die Trockenmasse des Pulvers, an freier oder salzartig gebundener Aminoverbindung verwendet, wobei die Summe aus Gelatinemenge und Menge an Aminoverbindung nicht mehr als 45 Gew.-%, vorzugsweise 40 Gew.-%, insbesondere 35 Gew.-%, betragen soll.

Die Verwendung von Aminosäuren und reduzierenden Zuckern für die Herstellung von pulverförmigen Formulierungen von fettlöslichen Vitaminen und deren Trocknungsprozess durch Hitzeeinwirkung ist zwar schon in der JA-AS 45-38348 (publiziert 1970) beschrieben, jedoch werden hierin nur Vitaminpulver auf Basis von Alkalisalzen von Casein, nicht aber von Gelatine, beschrieben. Da Casein jedoch im Gegensatz zu Gelatine keine thermoreversiblen Gele bildet, können hiermit nur sehr feinteilige Produkte erhalten werden, die z.B. für die Verwendung unter hydrothermaler Belastung nicht gut geeignet sind, da sie wasserdispergierbar sind.

Dagegen zeichnen sich die erfindungsgemäß erzeugten pulverförmigen Präparate durch eine ausgezeichnete Stabilität und Heißwasserunlöslichkeit aus. Diese Eigenschaften zeigen sich auch im Vergleich mit Pulvern, die einen hohen Gelatine-Anteil, aber keine Aminoverbindungen aufweisen und sind von besonderer Bedeutung bei Verwendung im Futtermittelbereich, da hier die Vitamin- und Carotinoid-haltigen Pulver chemischer, mechanischer und/oder hydrothermaler Belastung in Prämixen bzw. bei der Verarbeitung in Pellets, Extrudaten oder Tabletten ausgesetzt werden. Weiterhin muß erwähnt werden, daß bei den erfindungsgemäß erhaltenen Pulvern die Bioverfügbarkeit der eingeschlossenen Wirkstoffe voll aufrecht erhalten wird.

Die Aminoverbindungen können erfindungsgemäß auch zusammen mit einer basischen Alkali- oder Erdalkaliverbindung, wie einem Alkali- oder Erdalkalihydroxid, einem Erdalkalioxid oder einem Erdalkali- oder Alkalicarbonat eingesetzt werden. Es können aber auch die basischen Verbindungen anstelle der Aminoverbindungen Verwendung finden. Bei Verwendung von basischen Verbindungen ist es am zweckmäßigsten so zu verfahren, daß die hergestellte Dispersion nach der Zugabe der basischen Verbindung einen pH-Wert von 7,5 bis 10, vorzugsweise 8 bis 9,5, insbesondere 8 bis 9, aufweist.

Durch die thermische Behandlung des zunächst erhaltenen Pulvers wird die enthaltene Gelatine durch Reaktion von deren freien Aminogruppen mit den reduzierenden Zuckern (Maillard Reaktion) denaturiert und wird so wasserunlöslich. Dieser Effekt wird synergistisch verstärkt durch die Anwesenheit von den oben beschriebenen Aminoverbindungen, insbesondere von Aminocarbonsäuren, welche ein Vernetzen der Matrix durch Bildung von Polymeren unterstützen. Dies bedeutet, daß ein Teil der Gelatine, der gemäß den Verfahren des Standes der Technik notwendig wäre, durch jeweils wesentlich geringere Mengen an den beschriebenen Aminoverbindungen ersetzt werden kann.

Bei Zusatz von basischen Verbindungen werden vermutlich die Aminogruppen der Gelatine aktiviert, wodurch erklärbar wäre, daß auch hierbei eine Reduzierung der benötigten Gelatine-Menge möglich ist.

Ein weiterer Vorteil des erfindungsgemäßen Verfahrens liegt darin, daß die üblichen Vernetzungstemperaturen bei Anwesenheit der Aminoverbindungen und/oder der basischen Verbindungen herabgesetzt werden, d.h. daß eine Vernetzung bereits ab Temperaturen von 60°C möglich ist, während gemäß EP 285 682 Vernetzungstemperaturen von 90 bis 180°C, vorzugsweise 105 bis 150°C, benötigt werden. Beim erfindungsgemäßen Verfahren kann so die thermische Belastung der Wirkstoffe bei der Herstellung im Vergleich zu dem Verfahren gemäß EP 285 682 gemindert werden.

Zu den fettlöslichen Vitaminen zählen die Vitamine A, E, D und K sowie deren Mischungen. Sie können im Rahmen der Erfindung in Form von Vitaminlösungen in Ölen, als Provitamine sowie als reine Vitamine natürlichen oder synthetischen Ursprungs eingesetzt werden. Von besonderem Interesse sind Präparate von Vitamin A und dessen Derivaten, insbesondere von Vitamin-A-Acetat, Vitamin-A-Palmitat und Vitamin-A-Propionat sowie deren Mischungen.

Unter Carotinoiden werden Verbindungen verstanden wie β-Carotin, Apo-8'-carotinsäureethylester, Apo-8'-carotinal, Citranaxanthin, Canthaxanthin, Zeaxanthin, Astaxanthin, Lutein, Capsanthin und deren Mischungen.

Die Anteile an Vitaminen oder Carotinoiden betragen im allgemeinen etwa 5 bis 50 Gew.-%, vorzugsweise 10 bis 35 Gew.-%, bezogen auf die Trockenmasse der Pulver.

Für die Herstellung der Dispersion kann bei dem erfindungsgemäßen Verfahren Gelatine des A- und B-Typs in einem weiten Bloombereich eingesetzt werden. Mit besonderem Vorteil arbeitet man mit Gelatine mit einem Bloomwert von etwa 50 bis etwa 250.

Bei der Herstellung der Dispersion können alle reduzierenden Zucker oder Zuckersirupe mit Anteilen von reduzierenden Zukkern verwendet werden. Zu reduzierenden Zuckern zählen unter anderen Fructose, Glucose, Lactose, Maltose, Xylose, Arabinose, Ribose sowie Invertzucker (Glucose + Fructose), Honig, Fructose- und Glucose-Sirupe. Die Zucker verwendet man im allgemeinen in Mengen von etwa 3 bis 25 Gew.-%, bezogen auf die Trockenmasse.

Als organische Aminoverbindungen, die eine basische primäre Aminogruppe und zusätzlich entweder eine weitere Aminogruppe, eine Hydroxylgruppe, eine Alkoxygruppe oder eine Carboxylgruppe enthalten, kommen also aliphatische Diamine, wie Ethylendiamin oder Hexamethylendiamin; Alkanolamine, wie Ethanolamin, Propanolamin oder Butanolamin; Aminoether, wie 2-Methoxy-ethylamin oder 3-Methoxy-propylamin; oder Aminocarbonsäuren in freier oder salzartig gebundener Form in Betracht. Von besonderer Bedeutung ist die Verwendung von Aminocarbonsäuren, bzw. von deren Salzen.

Als Aminocarbonsäuren kommen alle natürlichen und synthetischen Aminosäuren in Form ihrer L-Form, D-Form oder als Racemat in Betracht. Die Aminocarbonsäuren können als α-, β- oder ω-Aminocarbonsäuren in freier Form oder als salzartige Verbindung mit Säuren oder Basen vorliegen. Genannt seien beispielsweise Glycin, α-Alanin, β-Alanin, Valin, γ-Aminobuttersäure, Leucin, Isoleucin, Tyrosin, Serin, Methionin, Arginin, Phenylalanin, Tryptophan oder Lysin oder aber Salze der genannten Aminosäuren.

Als Aminocarbonsäurekomponente können natürlich auch Gemische von freien oder salzartigen Aminocarbonsäuren verwendet werden, wie sie z.B. in Proteinhydrolysaten anfallen.

Besonders vorteilhaft ist die Verwendung von gut zugänglichen und dadurch billigen Aminocarbonsäuren bzw. deren Salzen, wie Lysinhydrochlorid oder Calcium- β-alaninat, insbesondere Calcium-β-alaninat.

Die Aminoverbindungen verwendet man im allgemeinen in Mengen von etwa 0,3 bis 20 Gew.-%, vorzugsweise 0,5 bis 10 Gew.-%, bezogen auf die Trockenmasse des Pulvers.

Die Gelatine verwendet man erfindungsgemäß in Mengen von 20 bis 35 Gew.-%, vorzugsweise 24 bis 34 Gew.-%, insbesondere 20 bis 30 Gew.-%, bezogen auf die Trockenmasse des Pulvers.

Vorteile gegenüber den bekannten Produkten ergeben sich bei den erfindungsgemäßen Trockenpulvern, bzw. bei deren Herstellung, wenn man so arbeitet, daß die Summe aus Gelatinemenge und der Menge an der organischen Aminoverbindung nicht mehr als 45 Gew.-%, vorzugsweise 40 Gew.-%, insbesondere 35 Gew.-% beträgt.

Zusätzlich zu den obligatorischen Bestandteilen werden der Dispersion mit Vorteil noch andere, bei der Herstellung von Wirkstofftrockenpulvern übliche Verbindungen, zugefügt.

Von besonderer Bedeutung für einen Einsatz der Trockenpulver als Futtermittelzusatz ist bei oxidationsempfindlichen Wirkstoffen ein Zusatz von Antioxidantien, wie Ethoxyquin, butyliertes Hydroxytoluol (BHT), butyliertes Hydroxyanisol (BHA) oder Tocopherol, sowie Stabilisatoren, wie Zitronensäure, Phosphorsäure oder Phytinsäure und deren Alkali- oder Erdalkalisalze, oder aber Komplexbildner, wie Ethylendiamintetraessigsäure (EDTA) oder Nitrilotriessigsäure (NTA).

Häufig werden der Emulsion aber auch Feuchthaltemittel, wie Glycerin, Sorbitol oder Polyethylenglykole oder auch zusätzliche Emulgatoren, wie Lecithin, zugefügt.

Darüberhinaus haben sich Zusätze, wie Stärke, insbesondere Maisstärke oder Maltodextrin oder Dickungsmittel, wie Gummiarabicum, Guargummi, Alginate und bestimmte abgebaute Stärken zur Einstellung der Viskosität der Emulsion als nützlich erwiesen.

Bezüglich näherer Details über Bedeutung, Art und Menge solcher Zusätze verweisen wir auf entsprechende Fachliteratur, beispielsweise auf die obengenannte Monographie "Fat-soluble Vitamins", Vol. 9, insbesondere Seiten 128 bis 133.

Zur Durchführung des erfindungsgemäßen Verfahrens geht man im allgemeinen so vor, daß man zur Herstellung der die Wirkstoffe enthaltenden Dispersion Gelatine in heißem Wasser (von 50 bis 70°C) zur Lösung bringt, zu dieser Lösung die Zucker, die Aminoverbindungen, die Vitamine und/oder Carotinoide, Stabilisatoren und die anderen üblichen Zusatzstoffe sowie ggf. noch zusätzlich Wasser addiert und das Ganze durch kräftiges Rühren bei erhöhter Temperatur dispergiert. Für die im letzten Verarbeitungsschritt erfolgende thermische Vernetzung des Pulvers sollte die fertige Dispersion in einem pH-Bereich von 4 bis 10 vorliegen, welcher durch Zugabe von Basen, wie NaOH, KOH, Ca(OH)₂, MgO, Soda oder NH₄OH eingestellt werden kann.

Die anschließende Weiterverarbeitung der Dispersion zu den erfindungsgemäßen Pulvern kann nach allen literaturbekannten Verfahren erfolgen.

Wegen der gewünschten Kornverteilung der Pulver (0,1 bis 0,6 mm Durchmesser) werden solche Verfahren bevorzugt, bei denen Vorkehrungen getroffen werden, daß die gelierten Tröpfchen der Dispersion voneinander getrennt bleiben, bis sich ihre Form stabilisiert hat.

Genannt seien beispielsweise das Verfahren gemäß EP-B1-74050, bei dem die Dispersion in hydrophobe Kieselsäure oder ein Metallsalz einer höheren Fettsäure versprüht wird oder aber das Verfahren gemäß EP-B1 285 682, bei dem die Dispersion in Stärkepulver versprüht wird. Es hat sich erwiesen, daß insbesondere bei Verfahren, die für Zubereitungen mit Gelatine-Gehalten unter 35 Gew.-% eingesetzt werden, die Versprühung mit hydrophober Kieselsäure als Pudermittel besonders vorteilhaft durchführbar ist.

Die nach den beschriebenen Verfahren erzeugten Pulver besitzen nach dem Trocknen einen Wassergehalt von weniger als 10 %, normalerweise weniger als 6 %. Die so erhaltenen pulverförmigen Präparate bestehen aus Teilchen mit gut ausgebildeter Oberfläche. Sie lösen sich in warmem Wasser von ca. 40°C rasch zu einer milchigen Dispersion.

Die thermische Härtung der getrockneten Pulver erfolgt durch Erhitzen auf Temperaturen von 60 bis 180°C, wobei der dabei erfolgende Vernetzungsprozeß mit steigender Temperatur immer schneller abläuft. Vorzugsweise wird die Vernetzung bei Temperaturen von 70 bis 130°C in einer Reaktionszeit von 5 Minuten bis 3 Stunden durchgeführt. Die so hergestellten Pulver sind unlöslich in kochendem Wasser und weisen eine ausgezeichnete Stabilität auf, wie der nachfolgende Stabilitätstest mit Vitamin-A-Acetat-Trockenpulver zeigt.

Mit Hilfe des erfindungsgemäßen Verfahrens können stabile heißwasserunlösliche Vitamin- und/oder Carotinoid-Trockenpulver hergestellt werden, die trotz Verwendung von nur etwa 20 bis 35 Gew.-%, bezogen auf die Trockenmasse des Pulvers, an Gelatine im Vergleich mit etwa 40 bis 45 Gew.-%, bezogen auf die Trockenmasse des Pulvers, gemäß dem Stand der Technik, eine mindestens gleich gute Stabilität aufweisen.

### Stabilitätstest

Verglichen wurde ein erfindungsgemäßes Vitamin-A-acetat-Trockenpulver auf Basis von Gelatine und Lysin (vgl. Ausführungsbeispiel 3) mit einem nur Gelatine enthaltenden, aber sonst gleich hergestellten Vitamin-A-acetat-Trockenpulver (vgl. Vergleichsbeispiel 4). Beide Trockenpulver wurden sowohl als nur getrocknetes (also unvernetztes) als auch als durch thermische Behandlung vernetztes Produkt dem im folgenden erläuterten sogenannten Prämixtest unter Streßbedingungen (40°C/70 % relative Luftfeuchigkeit) unterzogen.

Der Prämix hatte folgende Zusammensetzung:

| | |
|---|---|
| Weizengrießkleie | 60 % |
| Cholinchlorid (50 % auf SiO₂) | 30 % |
| Spurenelementmischung | 10 % |

Die Spurenelementmischung bestand aus:

| | |
|---|---|
| CuSO₄ · 5 H₂O | 37,43 % |
| FeSO₄ · 7 H₂O | 46,78 % |
| ZnO | 11,79 % |
| MnO | 3,61 % |
| CoCO₃ | 0,39 % |

In je 99 g des Prämix wurden je 1 g Vitamin-A-Trockenpulver eingemischt. Jeweils 4 g Mengen der fertigen Prämixgemische wurden bei 40°C und 70 % rel. Feuchte im Klimaschrank gelagert und am Anfang sowie nach 4 Wochen auf ihren Vitamin-A-Gehalt geprüft. Die noch gefundenen Vitamin-A-Gehalte sind in der nachstehenden Tabelle in % bezogen auf die Ausgangsgehalte angegeben.

Ein vergleichbarer Indikator für die Stabilität ist auch der enzymatisch nachweisbare Restzucker nach dem Vernetzen. Mit zunehmendem Vernetzungsgrad nimmt der nachweisbare Zuckergehalt des Pulvers ab und umgekehrt die Stabilität zu. Daher werden in der folgenden Tabelle auch die Restzuckerwerte angegeben. Weitere Beispiele für den Restzuckergehalt nach dem Vernetzen sind bei den Ausführungsbeispielen aufgeführt.

| | Trockenpulver gemäß Bsp. 3 | | Trockenpulver gemäß Bsp. 4 | |
|---|---|---|---|---|
| | a) unvernetzt | b) vernetzt | a) unvernetzt | b) vernetzt |
| Retention nach 4 Wochen | 64 % | 83 % | 60 % | 77 % |
| Restfructose | 9 % | 3,6 % | 9 % | 6,4 % |

Die Stabilitätsprüfung zeigt, daß thermisch vernetzte Vitamin-A-Trockenpulver deutlich stabiler sind als ihre unvernetzten Ausgangsprodukte. Darüber hinaus ist das erfindungsgemäße Vitamin-A-Trockenpulver (Ausführungsbeispiel 3 enthaltend 25 % Gelatine und 2,5 % Lysin), noch etwas stabiler als das Vitamin-A-Trockenpulver gemäß Vergleichsbeispiel 4 (40 % Gelatine, 0 % Lysin).

Beim Vergleich der Stabilitätsdaten von Trockenpulvern im zuvorbeschriebenen Prämix-Test wird auch ein deutlicher Stabilitätszuwachs beobachtet, wenn die Sprühdispersion mit basischen Verbindungen auf einen höheren pH-Wert eingestellt wird. Dies zeigt ein Vergleich der folgenden Beispiele 19 und 20.

### Beispiel 1

Zu 230 g Wasser wurden 85,2 g Gelatine A 100 Bloom gegeben und nach 30-minütigem Quellen durch Erhitzen auf 70°C in Lösung gebracht. Nach Zugabe von 42,8 g Fructosesirup (Zuckergehalt 70 %, davon 95 % Fructose in der Trockenmasse), wurden nacheinander 15 g Glycerin, 89,1 g Maisstärke, 7,5 g β-Alanin sowie 75,3 g Vitamin-A-Acetat (2,19 Millionen IE/g, hergestellt aus Vitamin-A-Acetat 2,9 Millionen IE/g und stabilisiert mit 100 mg Ethoxyquin und 14,5 mg BHT pro Million IE Vitamin A) hinzugegeben. Das Gemisch wurde nach Zugabe von weiteren 170 g Wasser durch kräftiges Rühren bei 60°C emulgiert. Die hergestellte Emulsion wurde bei einer Temperatur von 55°C mit einer Einstoffdüse bei 5,5 bis 6,5 bar in einem Sprühturm in eine Wolke hydrophober Kieselsäure gesprüht. Das noch feuchte Produkt wurde auf einem Wirbeltrockner bei Raumtemperatur auf eine Restfeuchte von 5,2 % getrocknet und von der überschüssigen Kieselsäure abgetrennt. Der enzymatisch bestimmte Fructosegehalt betrug 9,0 %. Anschließend wurden 10 g des erhaltenen Pulvers in einem rotierenden Aluminiumkolben, der in ein auf 120°C erhitztes Ölheizbad getaucht war, 20 Minuten (min) lang getempert. Das erhaltene braune Pulver besaß einen Vitamin-A-Gehalt von 560 300 IE/g bei einem Restfeuchtegehalt von 2,1 %. Die Restfructose wurde zu 2,4 % bestimmt.

10 g des noch nicht vernetzten Pulvers wurden alternativ für 24 min bei 100°C getempert. Das erhaltene braune Pulver war in kochendem Wasser nicht mehr dispergierbar (Partikel bleiben vollständig erhalten).

### Beispiele 2 bis 16

Auf die gleiche Art und Weise wie in Beispiel 1 wurde jeweils eine Emulsion mit den in der folgenden Tabelle angegebenen Inhaltsstoffen hergestellt, zu einem Pulver versprüht und getrocknet und anschließend für 20 min bei 120°C getempert. In allen Versuchen wurde ein Vitamin-A-Acetat enthaltend 2,19 Mill. IE/g verwendet, welches mit 100 mg Ethoxyquin und 14,5 mg BHT pro Mill. IE stabilisiert worden war. In allen Beispielen wurde Gelatine A 100 Bloom verwendet. In allen Beispielen, in denen Fructosesirup eingesetzt wird, handelt es sich um den in Beispiel 1 genauer beschriebenen Fructosesirup.

Die in der folgenden Tabelle angegebenen Gehalte an Gelatine sind Gewichtsprozente, bezogen auf das getrocknete Pulver. Sie wurden errechnet unter Berücksichtigung der in den Hilfsstoffen enthaltenen Wassermengen (Gelatine 12 %; Maisstärke 13 %, Zucker 30 %) sowie unter Berücksichtigung des Restwassergehalts und der Kieselsäurebepuderung, die zusammen etwa 7 Gew.-% ausmachen.

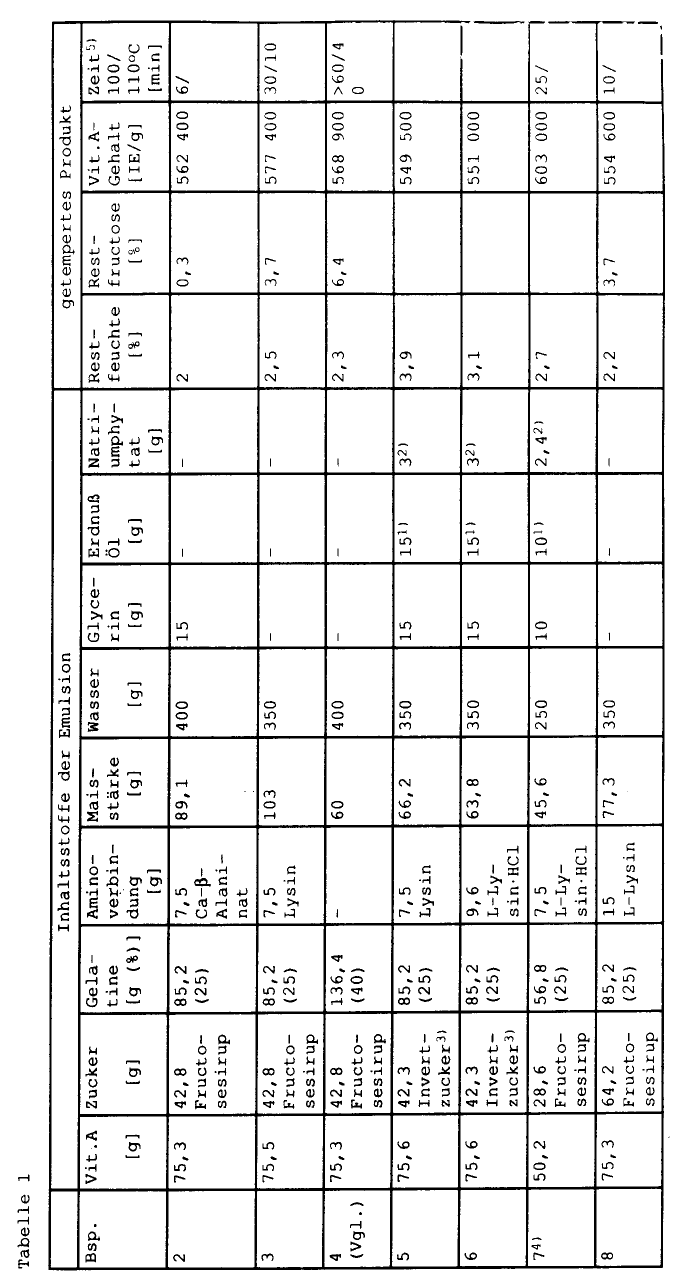

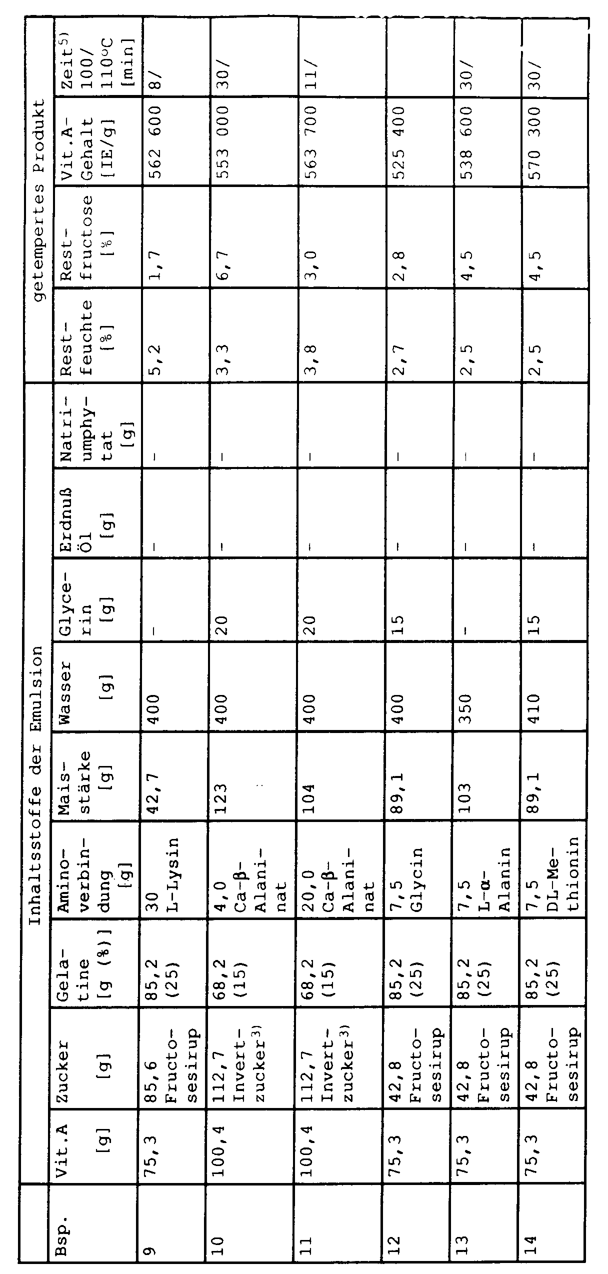

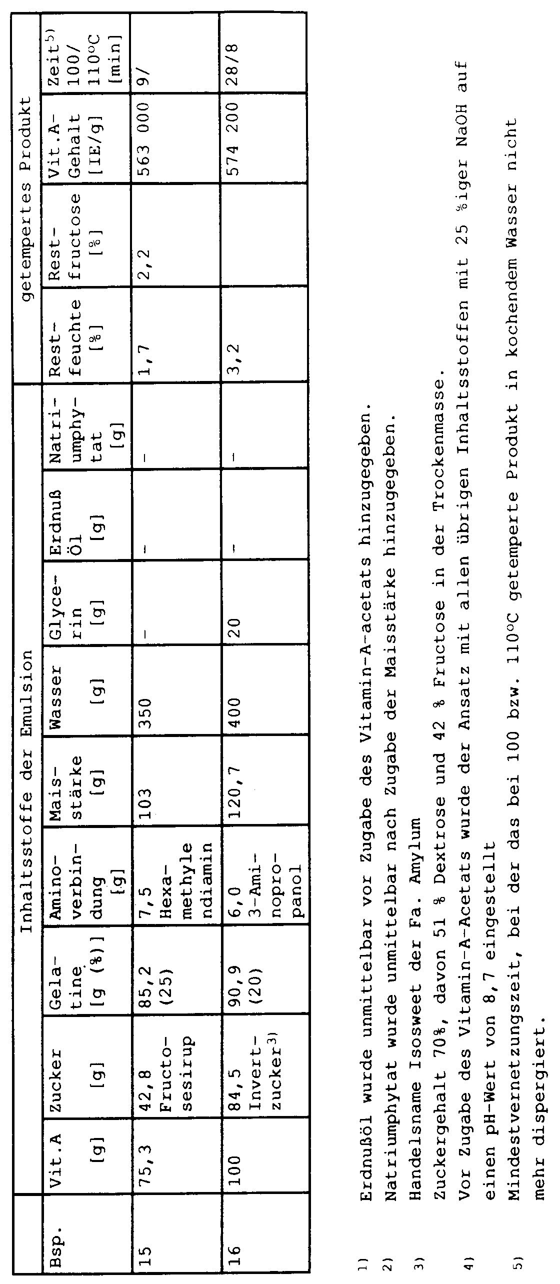

### Beispiel 17

In der gleichen Art und Weise wie in Beispiel 1 wurde eine Emulsion mit den nachstehend aufgeführten Inhaltsstoffen hergestellt, zu einem Pulver versprüht und getrocknet.

| | |
|---|---|
| Vitamin-A-Acetat 2,19 Mill. IE/g stabilisiert mit 100 mg Ethoxyquin und 14,5 mg BHT pro Mill. IE | 52,4 g |
| Vitamin D3 40 Mill. IE/g (in Vitamin-A-Acetat gelöst) | 0,55 g |
| Fructosesirup | 29,8 g |
| Gelatine A 100 Bloom | 59,3 g |
| Ca-β-Alaninat | 5,2 g |
| Maisstärke | 72,0 g |
| Wasser | 280,0 g |

Das erhaltene Produkt wurde für 20 min bei 120°C getempert. Die Restfeuchte betrug 4,1 %, der Restfructosegehalt 2,4 %, der Vitamin-A-Gehalt 558 200 IE/g und der Vitamin-D3-Gehalt 109 000 IE/g.

### Beispiel 18

In der gleichen Art und Weise wie in Beispiel 1 wurde eine Canthaxanthindispersion mit den nachstehend aufgeführten Inhaltsstoffen hergestellt, zu einem Pulver versprüht und getrocknet.

| | |
|---|---|
| Canthaxanthin mikronisiert | 37,0 g |
| Ethoxyquin | 11,0 g |
| Ascorbylpalmitat | 3,0 g |
| Gelatine B 200 Bloom | 94,3 g |
| Invertzucker³⁾ | 224,3 g |
| Ca-β-Alaninat | 17,0 g |
| Wasser | 613,0 g |

Die Restfeuchte des erhaltenen Pulvers betrug 7 %. Der enzymatisch festgestellt Fructosegehalt lag bei 16,0 % und Glucose bei 20,8 %.

Das Pulver wurde für 20 min bei 120°C getempert. Die Restfeuchte des getemperten Produkts betrug 4,2 %, die Restfructose und Glucose wurden zu 11 % und 4,6 % bestimmt. Der Canthaxanthingehalt des Pulvers betrug 12,2 %.

### Beispiele 19 bis 21

Zu 230 g Wasser wurden 99,9 g Gelatine A 100 Bloom gegeben und nach 30-minütigem Quellen durch Erhitzen auf 70°C in Lösung gebracht. Nach Zugabe von 61,9 g Invertzucker (Handelsname Isosweet der Fa. Amylum mit einem Zuckergehalt von 70 %, davon 51 % Dextrose und 42 % Fructose in der Trockenmasse) wurden nacheinander die in der Tabelle 2 angegebenen Mengen an Glyzerin, Maisstärke und Vitamin-A-Acetat (2,19 Millionen IE/g, hergestellt aus Vitamin-A-Acetat 2,9 Millionen IE/g und stabilisiert mit 100 mg Ethoxyquin und 14,5 mg BHT pro Million E Vitamin A) hinzugegeben. Das Gemisch wurde nach Zugabe von weiteren 140 g Wasser und der aus Tabelle 2 ersichtliche Menge einer 10 %igen wäßrigen NaOH-Lösung auf den in Tabelle 2 angegebenen pH-Wert eingestellt und durch kräftiges Rühren bei 60°C emulgiert.

Die so hergestellte Emulsion wurde bei einer Temperatur von 55°C mit einer Einstoffdüse bei 5,5 bis 6,5 bar in einem Sprühturm in einer Wolke hydrophober Kieselsäure gesprüht. Das noch feuchte Produkt wurde auf einem Wirbeltrockner bei Raumtemperatur auf eine Restfeuchte von 5,2 % getrocknet und von der überschüssigen Kieselsäure abgetrennt. Anschließend wurden 10 g des erhaltenen Pulvers in einem rotierenden Aluminiumkolben, der in ein auf 110°C erhitztes Ölheizbad getaucht war, für die in Tabelle 2 angegebene Zeit (Mindestvernetzungszeit, bei der das bei 110°C getemperte Produkt in kochendem Wasser nicht mehr dispergiert) getempert.

Die so erhaltenen Produkte wurden für 6 Wochen dem oben erläuterten sogenannten Prämix-Test unter Streßbedingungen (40°C/70 % reaktive Luftfeuchtigkeit) ausgesetzt und danach auf ihren Vitamin-A-Gehalt geprüft.

Ein Vergleich der Ergebnisse der Beispiele 19 und 21 mit dem Vergleichsbeispiel 20 (ohne Zugabe einer Base zur Emulsion) zeigt, daß durch Erhöhen des pH-Wertes der Sprühemulsion mit NaOH ein deutlicher Stabilitätszuwachs des Produktes sowie eine Verringerung der Mindestvernetzungszeit erreicht werden kann.

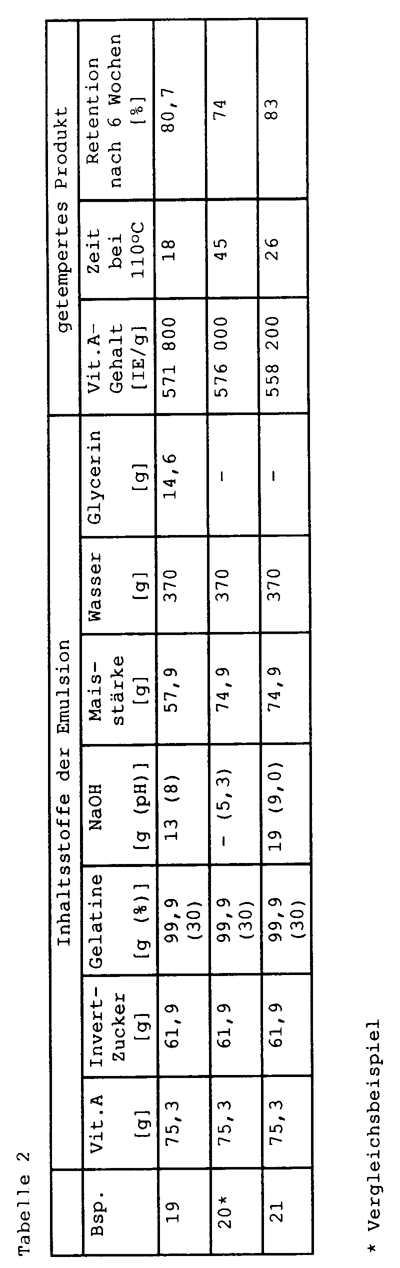

### Beispiel 22

Auf die gleiche Weise wie für die Beispiele 19 bis 21 beschrieben, wurde jeweils eine Emulsion der für Vergleichsbeispiel 20 angegebenen Zusammensetzung hergestellt und anschließend mit der aus Tabelle 3 ersichtlichen Base auf den aus Tabelle 3 ersichtlichen pH-Wert eingestellt und für die analog den Beispielen 19 bis 21 hergestellten Trockenpulver die Mindestvernetzungszeit bei 120°C bestimmt.

**Tabelle 3**

| Rezeptur der Emulsion | Basenzusatz | pH-Wert der Sprühemulsion | Mindestvernetzungszeit bei 120°C [min] |
|---|---|---|---|
| a) wie Bsp. 20 | - | 5,3 | 26 |
| b) wie Bsp. 20 | NaOH | 9,0 | 11 |
| c) wie Bsp. 20 | KOH | 9,0 | 10 |
| d) wie Bsp. 20 | MgO | 9,0 | 14 |
| e) wie Bsp. 20 | Ca(OH)₂ | 9,0 | 11 |

Durch eine Erhöhung des pH-Wertes der Emulsion vor dem Versprühen durch verschiedene Basen kann die Mindestvernetzungszeit für die Trockenpulver deutlich reduziert werden.

## Patentansprüche

1. Stabile heißwasserunlösliche Trockenpulver, bei welchen ein oder mehrere fettlösliche Vitamine und/oder ein oder mehrere Carotinoide in eine Matrix auf Basis von Gelatine eingebettet sind, erhältlich durch
a) Herstellung einer wäßrigen Dispersion enthaltend im wesentlichen diese fettlöslichen Wirkstoffe, reduzierende Zucker und als filmbildendes Kolloid Gelatine in Mengen von 20 bis 35 Gew.-%, bezogen auf die Trockenmasse des Pulvers, in Kombination mit physiologisch verträglichen organischen Aminoverbindungen, die eine basische primäre Aminogruppe und zusätzlich entweder eine weitere Aminogruppe, eine Hydroxylgruppe, eine Alkoxygruppe oder eine Carboxylgruppe enthalten, in freier oder in salzartig gebundener Form und/oder in Kombination mit soviel einer basischen Alkali- oder Erdalkaliverbindung, daß die Dispersion einen pH-Wert von 7,5 bis 10 aufweist,
b) Überführung dieser Dispersion in trockene, pulverförmige Vitamin- und/oder Carotinoidpräparate und
c) thermische Härtung des Pulvers bei Temperaturen von 60 bis 180°C.

2. Stabile heißwasserunlösliche Trockenpulver gemäß Anspruch 1, dadurch gekennzeichnet, daß man die Gelatine in Mengen von 20 bis 35 Gew.-%, bezogen auf die Trockenmasse des Pulvers und die freie oder salzartig gebundene Aminoverbindung in Mengen von 0,3 bis 20 Gew.-%, bezogen auf die Trockenmasse des Pulvers verwendet, wobei die Summe aus Gelatinemenge und Menge der Aminoverbindung nicht mehr als 45 Gew.-% betragen soll.

3. Stabile heißwasserunlösliche Trockenpulver gemäß einem der Ansprüche 1 oder 2, dadurch gekennzeichnet, daß man als filmbildendes Kolloid Gelatine in Kombination mit einer oder mehreren freien oder salzartig gebundenen Aminocarbonsäuren verwendet.

4. Stabile heißwasserunlösliche Trockenpulver gemäß Anspruch 3, dadurch gekennzeichnet, daß man als salzartig gebundene Aminocarbonsäure Calcium-β-Alaninat verwendet.

5. Stabile heißwasserunlösliche Trockenpulver gemäß Anspruch 1, dadurch gekennzeichnet, daß man die Gelatine in Mengen von 24 bis 34 Gew.-%, bezogen auf die Trockenmasse des Pulvers in Kombination mit soviel eines Alkali- oder Erdalkalihydroxids verwendet, daß die Dispersion einen pH-Wert von 8 bis 9,5 aufweist.

6. Verfahren zur Herstellung von Stabilen heißwasserunlöslichen Trockenpulvern, welche ein oder mehrere fettlösliche Vitamine und/oder ein oder mehrere Carotinoide enthalten, welches folgende Verfahrensschritte beinhaltet:
a) Herstellung einer wäßrigen Dispersion enthaltend im wesentlichen diese fettlöslichen Wirkstoffe, filmbildende Kolloide und reduzierende Zucker,
b) Überführung dieser Dispersion in trockene, pulverförmige Vitamin- und/oder Carotinoidpräparate und
c) thermische Härtung des Pulvers bei Temperaturen von 60 bis 180°C,
dadurch gekennzeichnet, daß man als filmbildendes Kolloid Gelatine in Mengen von 20 bis 35 Gew.-%, bezogen auf die Trockenmasse des Pulvers, in Kombination mit einer oder mehreren physiologisch verträglichen freien oder salzartig gebundenen Aminoverbindungen, die eine basische primäre Aminogruppe und zusätzlich entweder eine weitere Aminogruppe, eine Hydroxylgruppe, eine Alkoxygruppe oder eine Carboxylgruppe enthalten, und/oder in Kombination mit soviel einer basischen Alkali- oder Erdalkaliverbindung verwendet, daß die Dispersion einen pH-Wert von 7,5 bis 10 aufweist.

7. Verfahren gemäß Anspruch 6, dadurch gekennzeichnet, daß man als filmbildendes Kolloid Gelatine in Mengen von 20 bis 35 Gew.-%, bezogen auf die Trockenmasse des Pulvers, in Kombination mit 0,3 bis 20 Gew.-%, bezogen auf die Trockenmasse des Pulvers, an der oder den freien oder salzartig gebundenen Aminoverbindungen verwendet, wobei die Summe aus Gelatinemenge und Menge an Aminoverbindung nicht mehr als 45 Gew.-% betragen soll.

8. Verfahren gemäß Anspruch 6, dadurch gekennzeichnet, daß man als filmbildendes Kolloid Gelatine in Kombination mit Lysin, oder β-Alanin oder einem ihrer Salze verwendet.

9. Verfahren gemäß Anspruch 6, dadurch gekennzeichnet, daß man als filmbildendes Kolloid Gelatine in Kombination mit Calcium-β-Alaninat verwendet.

10. Verfahren gemäß Anspruch 6, dadurch gekennzeichnet, daß man als filmbildendes Kolloid Gelatine in Kombination mit soviel eines Alkali- oder Erdalkalihydroxids verwendet, daß die Dispersion einen pH-Wert von 8 bis 9,5 aufweist.

## Claims

1. A stable dry powder which is insoluble in hot water and in which one or more fat-soluble vitamins and/or one or more carotenoids are embedded in a gelatin-based matrix, obtainable by
a) preparing an aqueous dispersion containing essentially these fat-soluble active substances, reducing sugars and, as film-forming colloid, gelatin in amounts of from 20 to 35% of the weight of the powder dry matter in combination with physiologically tolerated organic amino compounds which contain a basic primary amino group and, in addition, either another amino group, a hydroxyl group, an alkoxy group or a carboxyl group, in free form or in a form bonded in the manner of a salt and/or in combination with an amount of a basic alkali metal or alkaline earth metal compound such that the dispersion has a pH of from 7.5 to 10,
b) converting this dispersion into dry vitamin and/or carotenoid products in powder form and
c) thermally curing the powder at from 60 to 180°C.

2. A stable dry powder which is insoluble in hot water as claimed in claim 1, wherein the gelatin is used in amounts of from 20 to 35% of the weight of the powder dry matter, and the amino compound which is free or bonded in the manner of a salt is used in amounts of from 0.3 to 20% by weight of the powder dry matter, the total of the amount of gelatin and the amount of the amino compound not exceeding 45% by weight.

3. A stable dry powder which is insoluble in hot water as claimed in either of claims 1 or 2, wherein gelatin in combination with one or more amino carboxylic acids which are free or bonded in the manner of a salt is used as film-forming colloid.

4. A stable dry powder which is insoluble in hot water as claimed in claim 3, wherein calcium β-alaninate is used as amino carboxylic acid which is bonded in the manner of a salt.

5. A stable dry powder which is insoluble in hot water as claimed in claim 1, wherein the gelatin is used in amounts of from 24 to 34% of the weight of the powder dry matter in combination with an amount of an alkali metal or alkaline earth metal hydroxide such that the dispersion has a pH of from 8 to 9.5.

6. A process for preparing stable dry powders which are insoluble in hot water and which contain one or more fat-soluble vitamins and/or one or more carotenoids, which comprises the following steps:
a) preparing an aqueous dispersion containing essentially these fat-soluble active substances, film-forming colloids and reducing sugars,
b) converting this dispersion into dry vitamin and/or carotenoid products in powder form and
c) thermally curing the powder at from 60 to 180°C,
wherein gelatin in amounts of from 20 to 35% of the weight of the powder dry matter in combination with one or more physiologically tolerated amino compounds which are free or bonded in the manner of a salt and which contain a basic primary amino group and, in addition, either another amino group, a hydroxyl group, an alkoxy group or a carboxyl group, and/or in combination with an amount of a basic alkali metal or alkaline earth metal compound such that the dispersion has a pH of from 7.5 to 10 is used as film-forming colloid.

7. A process as claimed in claim 6, wherein gelatin is used in amounts of from 20 to 35% of the weight of the powder dry matter, in combination with from 0.3 to 20% by weight, based on the powder dry matter, of the amino compound or compounds which is/are free or bonded in the manner of a salt, the total of the amount of gelatin and the amount of amino compound not exceeding 45% by weight, as film-forming colloid.

8. A process as claimed in claim 6, wherein gelatin in combination with lysine or β-alanine or one of their salts is used as film-forming colloid.

9. A process as claimed in claim 6, wherein gelatin in combination with calcium β-alaninate is used as film-forming colloid.

10. A process as claimed in claim 6, wherein gelatin, in combination with an amount of an alkali metal or alkaline earth metal hydroxide such that the dispersion has a pH of from 8 to 9.5, is used as film-forming colloid.

## Revendications

1. Poudre sèche stable, insoluble dans l'eau chaude, dans laquelle une ou plusieurs vitamines liposolubles et/ou un ou plusieurs caroténoïdes sont enrobés dans une matrice à base de gélatine, pouvant être obtenue par
a) préparation d'une dispersion aqueuse contenant essentiellement ces substances actives liposolubles, un sucre réducteur et, comme colloïde filmogène,de la gélatine en une quantité de 20 à 35 % en poids, par rapport à la masse sèche de la poudre, en combinaison avec des composés amino organiques physiologiquement acceptables, qui contiennent un groupe amino primaire basique et en outre un autre groupe amino, un groupe hydroxyle, un groupe alcoxy ou un groupe carboxyle, sous une forme libre ou sous forme liée de type sel et/ou en combinaison avec un composé alcalin ou alcalino-terreux basique en une quantité telle que la dispersion présente un pH de 7,5 à 10,
b) conversion de cette dispersion en une préparation sèche, pulvérulente, de vitamine et/ou de caroténoïde et
c) durcissement thermique de la poudre à des températures de 60 à 180°C.

2. Poudre sèche stable, insoluble dans l'eau chaude selon la revendication 1, caractérisée en ce qu'on utilise la gélatine en des quantités de 20 à 35 % en poids, par rapport à la masse sèche de la poudre et on utilise le composé amino libre ou lié sous forme de sel en des quantités de 0,3 à 20 % en poids, par rapport à la masse sèche de la poudre, tandis que la somme de la quantité de gélatine et de la quantité de composé amino ne doit pas dépasser 45 % en poids.

3. Poudre sèche stable, insoluble dans l'eau chaude, selon l'une des revendications 1 ou 2, caractérisée en ce qu'on emploie, comme colloïde filmogène, de la gélatine en combinaison avec un ou plusieurs acides aminocarboxyliques libres ou sous forme de sel.

4. Poudre sèche stable, insoluble dans l'eau chaude, selon la revendication 3, caractérisée en ce qu'on emploie, comme acide aminocarboxylique lié sous forme de sel, du β-alaninate de calcium.

5. Poudre sèche stable, insoluble dans l'eau chaude, selon la revendication 1, caractérisée en ce qu'on utilise la gélatine en des quantités de 24 à 34 % en poids, par rapport à la masse sèche de la poudre, en combinaison avec assez d'un hydroxyde alcalin ou alcalino-terreux pour que la dispersion présente un pH de 8 à 9,5.

6. Procédé pour la préparation de poudres sèches stables, insolubles dans l'eau chaude, qui contiennent une ou plusieurs vitamines liposolubles et/ou un ou plusieurs caroténioïdes, ledit procédé comportant les étapes opératoires suivantes:
a) préparation d'une dispersion aqueuse contenant pour l'essentiel ces agents actifs liposolubles, des colloïdes filmogènes et des sucres réducteurs,
b) transformation de cette dispersion en préparations sèches, pulvérulentes, de vitamines et/ou de caroténoïdes et
c) durcissement thermique de la poudre à des températures de 60 à 180°C,
caractérisé en ce qu'on emploie, comme colloïde filmogène, de la gélatine en des quantités de 20 à 35 % en poids, par rapport à la masse sèche de la poudre, en combinaison avec un ou plusieurs composés amino physiologiquement acceptables, libres ou liés sous forme de sel, qui contiennent un groupe amino primaire basique et en outre un autre groupe amino, un groupe hydroxyle, un groupe alcoxy ou un groupe carboxyle, et/ou en combinaison avec assez d'un composé alcalin ou alcalino-terreux basique pour que la dispersion présente un pH de 7,5 à 10.

7. Procédé selon la revendication 6, caractérisé en ce qu'on emploie comme colloïde filmogène de la gélatine en des quantités de 20 à 35 % en poids, par rapport à la masse sèche de la poudre, en combinaison avec 0,3 à 20 % en poids, par rapport à la masse sèche de la poudre, du ou des composés amino libres ou liés sous forme de sel, tandis que la somme de la quantité de gélatine et de la quantité de composé amino ne doit pas dépasser 45 % en poids.

8. Procédé selon la revendication 6, caractérisé en ce qu'on emploie, comme colloïde filmogène, de la gélatine en combinaison avec de la lysine, ou de la β-alanine ou un de ses sels.

9. Procédé selon la revendication 6, caractérisé en ce qu'on emploie, comme colloïde filmogène, de la gélatine en combinaison avec du β-alaninate de calcium.

10. Procédé selon la revendication 6, caractérisé en ce qu'on emploie, comme colloïde filmogène, de la gélatine en combinaison avec assez d'un hydroxyde alcalin ou alcalino-terreux pour que la dispersion présente un pH de 8 à 9,5.
